# EUROPEAN PATENT SPECIFICATION

(11) **EP 3 070 711 B1**
(45) Date of publication and mention of the grant of the patent: **21.03.2018**
(21) Application number: 14860083.6
(22) Date of filing: 10.11.2014
(51) Int. Cl.: G10L 15/00, B60R 25/24, B60R 25/25, E05B 49/00, E05F 15/603, G10L 17/00, H04R 1/00, H04R 1/08, H04R 19/00, B60K 28/06, G10L 15/30

(54) **SMART ENTRY SYSTEM**
INTELLIGENTES EINGABESYSTEM
SYSTÈME D'ENTRÉE INTELLIGENT

(30) Priority: 11.11.2013 JP 2013233185
(43) Date of publication of application: 21.09.2016
(73) Proprietor: Panasonic Intellectual Property Management Co., Ltd., Osaka-shi, Osaka 570-6207 (JP)
(72) Inventor: OGURA, Hiroshi, Osaka-shi, Osaka 540-6207 (JP); TAKESHI, Futoshi, Osaka-shi, Osaka 540-6207 (JP); DOI, Kazumoto, Osaka-shi, Osaka 540-6207 (JP)
(74) Representative: Grünecker Patent- und Rechtsanwälte PartG mbB
(86) International application number: PCT/JP2014/005640
(87) International publication number: WO 2015/068403

(56) References cited:
- DE-U1-202008 005 694
- GB-A- 2 396 730
- JP-A- H0 678 040
- JP-A- 2000 080 828
- JP-A- 2000 305 586
- JP-A- 2000 305 586
- JP-A- 2003 114 694
- JP-A- 2003 114 694
- JP-A- 2006 025 079
- JP-A- 2006 025 079
- JP-A- 2009 515 239
- JP-A- 2012 172 367
- US-A1- 2002 084 130
- US-A1- 2003 130 852
- US-A1- 2005 272 477
- US-A1- 2007 080 931
- US-A1- 2012 075 091

## Description

### TECHNICAL FIELD

The present invention relates to a smart entry system and, more particularly, to a handsfree vehicular smart entry system that makes it possible to open and close a vehicle door, for example, without using a hand.

### BACKGROUND ART

In recent years, smart entry systems having a smart entry function have been put into practical use. For example, there is a smart entry function that unlocks the doors automatically when the driver merely approaches a vehicle, and locks the doors automatically when the driver merely leaves the vehicle. In this case, more specifically, the doors are unlocked automatically when a portable unit has entered an effective area formed around the vehicle, and are locked automatically when the portable unit has exited the effective area. As for its smart entry mechanism, each of the vehicle and a smart entry device emits radio waves. When the distance between the vehicle and the smart entry device has become so short that they can communicate with each other, a registered ID of the smart entry device is subjected to authentication processing and the vehicle doors are unlocked if the registered ID is found legitimate. Smart entry systems of this kind are disclosed in Patent documents 1 and 2, for example.

On the other hand, in smart entry systems of this kind, the driver cannot open an electromotive slide door of a vehicle if neither of his or her hands is usable. In view of this, a smart entry system is disclosed in Patent document 3 that opens a door in a handsfree manner when a prescribed portion of the vehicle has received impact. Another smart entry system is disclosed in Patent document 4 that opens a door utilizing a laser beam in a handsfree manner.

Patent document 5 discloses a smart entry system that opens and closes a door in a handsfree manner using a microphone that is provided in a vehicular apparatus installed in the vehicle.

Patent document 6 discloses a security-enhanced smart entry system that allows only an identifiable person to use it by giving a fingerprint authentication function to a smart entry portable unit.

### Prior Art Documents

### Patent Documents

Patent document 1: Japanese Patent No. 5,161,476
Patent document 2: Japanese Patent No. 5,200,799
Patent document 3: JP-A-2004-316231
Patent document 4: JP-A-2007-162459
Patent document 5: JP-A-2012-172367
Patent document 6: JP-A-2007-238058

### SUMMARY OF THE INVENTION

### Problems to Be Solved by the Invention

However, among the conventional handsfree smart entry systems, the system disclosed in Patent document 3 may damage the vehicle (e.g., form a scratch) because it requires an act of, for example, kicking a side sill that is a bottom edge of a vehicle slide door using a foot, for example. In addition, if the driver is holding a load, kicking with a foot is a dangerous act because it may have the driver lose his or her balance.

The system of using a laser beam that is disclosed in Patent document 4 is not only dangerous because the driver needs to interrupt a laser beam using a foot, for example, and hence is prone to lose his or her balance but also not convenient to use because an area to be illuminated with a laser beam is restricted.

The smart entry system disclosed in Patent document 5 in which a microphone is provided in a vehicular apparatus installed in a vehicle also has problems that a voice of a talker may be difficult to pick up when the microphone is installed at an improper position and hence a space from which the system can be activated is restricted. Furthermore, where a microphone is installed in a vehicle, the sound pressure is reduced by the vehicle body (made of a steel plate, for example). As a result, it is difficult to pick up a voice with the microphone and the smart entry system is made substantially impossible to activate.

The system disclosed in Patent document 6 in which an individual is authenticated using fingerprint data is not handsfree because it requires reading of the fingerprint data with a portable unit.

The present invention has been made in view of the above problems, and an object of the invention is therefore to provide a smart entry system that can be activated in a handsfree manner from any place even in a state that the driver, or the like, is holding a load and neither of his or her hands is usable, without the driver's losing his or her balance because of an act of, for example, lifting up a foot.

This object is solved by the present invention as claimed in the appended independent claim. Particular embodiments of the present invention are defined by the appended dependent claims.

### Means for Solving the Problems

To solve the above problems, the inventors have made, as described below, an aspect of the invention relating to the structure of a portable unit used in a smart entry system and a smart entry system using it. Each aspect of the invention will be described below concisely.

According to a comparative example useful for understanding the present invention, it is provided a smart entry system which includes a portable unit configured to transmit and receive a signal, and a vehicular apparatus installed in a vehicle and configured to perform at least part of locking controls and unlocking controls of the vehicle by wirelessly communicating authentication signals between the portable unit and the vehicular apparatus bidirectionally, wherein the portable unit includes at least one hole through which a sound pressure is input ,a sound conversion element which is disposed behind the hole and converts the sound pressure into an electrical signal, and a voice recognition device which processes the electrical signal obtained by the conversion using a predetermined particular spoken voice as a voice key; and wherein at least a portion of the vehicle operates in response to input of the voice key.

This configuration makes it possible to provide a smart entry system that allows the driver, for example, to cause at least the portion of the vehicle to operate in a handsfree manner even in a state that he or she is holding a load and neither of his or her hands is usable, without losing his or her balance because of an act of, for example, lifting up a foot, and that can be activated from any place.

A further comparative example provides a smart entry system which is based on the above configuration and is characterized in that the operation of at least the portion of the vehicle is opening/closure of a door of the vehicle.

This configuration makes it possible to provide a smart entry system that allows the driver, for example, to open or close a vehicle door in a handsfree manner even in a state that he or she is holding a load and neither of his or her hands is usable, without losing his or her balance because of an act of, for example, lifting up a foot, and that can be activated from any place.

A further example provides a smart entry system which is based on the above configuration and is characterized in that the operation of at least the portion of the vehicle is opening/closure of a trunk of the vehicle.

This configuration makes it possible to provide a smart entry system that allows the driver, for example, to open or close a vehicle trunk in a handsfree manner even in a state that he or she is holding a load and neither of his or her hands is usable, without losing his or her balance because of an act of, for example, lifting up a foot, and that can be activated from any place.

This configuration also makes it possible to flash vehicle headlights, blow a horn, or cause a like operation in a handsfree manner.

A further comparative example provides a smart entry system which is based on the above configuration and is characterized in that the predetermined, particular spoken voice which is used as the voice key is made effective only when spoken by a particular talker.

This configuration enables handsfree personal authentication.

A further comparative example provides a smart entry system which is based on the above configuration and is characterized in that a sound shield member is interposed between the hole of the portable unit and the sound conversion element.

Since the sound shield member prevents entrance of a disturbance sound pressure, this configuration makes it possible to transmit a necessary sound pressure of a voice, for example, to the sound conversion element efficiently.

A further comparative example provides a smart entry system which is based on the above configuration and is characterized in that a water-proof film or a planar fabric having a water-repelling function is disposed in front of the hole of the portable unit.

Since entrance of, for example, water droplets such as raindrops into the sound conversion element is prevented, this configuration makes it possible to provide a portable unit and a smart entry system that are hard to fail.

A further comparative example provides a smart entry system which is based on the above configuration and is characterized in that a speaker is provided in the portable unit.

This configuration makes it possible to instruct a talker verbally from near him or her using the speaker of the portable unit.

A further comparative example provides a smart entry system which is based on the above configuration and is characterized in that a speaker is provided in the vehicle.

This configuration makes it possible to instruct a talker verbally without the need for installing a speaker in the portable unit.

A further comparative example provides a smart entry system which is based on the above configuration and is characterized in that a buzzer is provided in the vehicle.

This configuration makes it possible to notify nearby persons of an approaching door or trunk opening or closing operation before doing it.

A further comparative example provides a smart entry system which is based on the above configuration and is characterized in having a function of urging a talker to speak using the speaker.

Since a verbal instruction is made that urges a talker to speak, this configuration allows the talker to speak in a dialogue-type scheme and thereby increases the convenience of the talker.

A further comparative example provides a smart entry system which is based on the above configuration and is characterized in having a function of notifying, by the speaker, the talker that the voice key has been input correctly if a voice that is spoken by the talker after urging the talker to speak using the speaker is the same as the predetermined voice key.

With this configuration, a voice spoken by a talker is recognized correctly and hence the talker can wait for the next operation of the vehicle at ease.

A further comparative example provides a smart entry system which is based on the above configuration and is characterized in having a function of notifying, by a buzzer, the talker that the voice key has been input correctly if a voice that is spoken by the talker after urging the talker to speak using the speaker is the same as the predetermined voice key.

With this configuration, a voice spoken by a talker is recognized correctly and hence the talker can wait for the next operation of the vehicle at ease.

A further comparative example provides a smart entry system which is based on the above configuration and is characterized in having a function of urging a talker to speak by flashing lights of the vehicle by an electrical signal of the vehicular apparatus installed in the vehicle.

Since a verbal instruction is made that urges a talker to speak, this configuration allows the talker to speak in a dialogue-type scheme and thereby increases the convenience of the talker.

A further comparative example provides a smart entry system which is based on the above configuration and is characterized in that the portable unit includes a tie-pin-shaped clip.

With this configuration, if the tie-pin-shaped clip is attached to a chest pocket, for example, of clothes, a phenomenon can be prevented that a friction sound is produced by friction between the portable unit and a cloth when the portable unit is put in a bag, a trouser pocket, or the like. As a result, a voice spoken by a talker can be acquired without a noise.

A further comparative example provides a smart entry system which is based on the above configuration and is characterized in that the portable unit includes a U-shaped clip.

With this configuration, if the U-shaped clip is attached to a chest pocket, for example, of clothes, a phenomenon can be prevented that a friction sound is produced by friction between the portable unit and a cloth when the portable unit is put in a bag, a trouser pocket, or the like. As a result, a voice spoken by a talker can be acquired without a noise.

A further comparative example provides a smart entry system which is based on the above configuration and is characterized in that an alcohol detector is provided in the portable unit; and that the smart entry system has a function of disregarding a spoken voice of any voice key if the alcohol detector detects alcohol.

This configuration makes it possible to prevent drunken driving.

A further comparative example provides a smart entry system which is based on the above configuration and is characterized in that the portable unit has a non-contact charging function.

This configuration makes it possible to prevent a system shutdown due to sudden running-out of the battery of the portable unit.

The invention provides a smart entry system which includes a portable unit configured to transmit and receive a signal, a vehicular apparatus installed in a vehicle, and a headset configured to perform a wireless communication with the portable unit, and to perform at least part of locking controls and unlocking controls on the vehicle by wirelessly communicating authentication signals between the portable unit and the vehicular apparatus bidirectionally, wherein the headset includes at least one hole through which a sound pressure is input, a sound conversion element which is disposed behind the hole and converts the sound pressure into an electrical signal, and a voice recognition device which processes the electrical signal obtained by the conversion using a predetermined particular spoken voice as a voice key, wherein the headset transmits an input signal of the voice key to the portable unit by a wireless communication; and wherein the portable unit instructs the vehicular apparatus on at least part of operations of the vehicle by radio.

This configuration allows a voice spoken by a talker to travel to the portable unit even in a situation that a friction sound is produced by friction between the portable unit and a cloth because, for example, the portable unit is put in a bag, a trouser pocket, or the like, irrespective of the generation of the friction sound. As a result, a highly convenient, handsfree smart entry system can be provided.

The invention provides a smart entry system which is based on the above configuration and is characterized in that the instruction on the at least part of the operations of the vehicle is an opening/closure instruction for a door of the vehicle.

This configuration makes it possible to provide a smart entry system that allows the driver, for example, to open or close a vehicle door in a handsfree manner even in a state that he or she is holding a load and neither of his or her hands is usable, without losing his or her balance because of an act of, for example, lifting up a foot, and that can be activated from any place.

The invention provides a smart entry system which is based on the above configuration and is characterized in that the instruction on the at least part of the operations of the vehicle is an opening/closure instruction for a trunk of the vehicle.

This configuration makes it possible to provide a smart entry system that allows the driver, for example, to open or close a vehicle trunk in a handsfree manner even in a state that he or she is holding a load and neither of his or her hands is usable, without losing his or her balance because of an act of, for example, lifting up a foot, and that can be activated from any place.

This configuration also makes it possible to flash vehicle headlights, blow a horn, or cause a like operation in a handsfree manner.

The invention provides a smart entry system which is based on the above configuration and is characterized in that a speaker is provided in the headset; and the smart entry system has a function of urging a talker to speak by the speaker.

Since a verbal instruction is made that urges a talker to speak, this configuration allows the talker to speak in a dialogue-type scheme and thereby increases the convenience of the talker.

The invention provides a smart entry system which is based on the above configuration and is characterized in that a sound shield member is interposed between the hole of the headset and the sound conversion element.

Since the sound shield member prevents entrance of a disturbance sound pressure, this configuration makes it possible to transmit a necessary sound pressure of a voice, for example, to the sound conversion element efficiently.

The invention provides a smart entry system which is based on the above configuration and is characterized in that a water-proof film or a planar fabric having a water-repelling function is disposed in front of the hole of the headset.

Since entrance of, for example, water droplets such as raindrops into the sound conversion element is prevented, this configuration makes it possible to provide a portable unit and a smart entry system that are hard to fail.

The invention provides a smart entry system which is based on the above configuration and is characterized in that an alcohol detector is provided in the headset, and the smart entry system has a function of disregarding a spoken voice of any voice key if the alcohol detector detects alcohol.

This configuration makes it possible to prevent drunken driving.

The invention provides a smart entry system which is based on the above configuration and is characterized in that the predetermined, particular spoken voice which is used as the voice key is made effective only when spoken by a particular talker.

This configuration enables handsfree personal authentication.

### Advantages of the Invention

The invention can provide a smart entry system that can be activated in a handsfree manner from any place even in a state that the driver, for example, is holding a load and neither of his or her hands is usable, without the driver's losing his or her balance. The use of the smart entry system according to the invention makes it possible to provide a portable unit for a smart entry system that is highly resistant to environment-related factors such as rain. Furthermore, the smart entry system according to the invention enables handsfree personal authentication.

### BRIEF DESCRIPTION OF THE DRAWINGS

[Fig. 1] Fig. 1 is a block diagram showing the configuration of a smart entry system according to a first embodiment of the present invention.
[Fig. 2] Fig. 2 is a front view showing an appearance of a portable unit of the smart entry system according to the first embodiment of the invention.
[Fig. 3] Figs. 3(a)-3(d) are appearance diagrams showing an example acoustic transducer that is disposed inside the portable unit of the smart entry system according to the first embodiment of the invention.
[Fig. 4] Fig. 4 is an appearance perspective view showing an example sound conversion module which is disposed inside the portable unit of the smart entry system according to the first embodiment of the invention.
[Fig. 5] Figs. 5(a) and 5(b) are a sectional view of the portable unit of the smart entry system according to the first embodiment of the invention.
[Fig. 6] Fig. 6 is a sectional view of the portable unit of the smart entry system according to the first embodiment of the invention.
[Fig. 7] Fig. 7 is a block diagram showing a schematic configuration for charging the portable unit of the smart entry system according to the first embodiment of the invention by non-contact charging.
[Fig. 8] Fig. 8 shows an example manner of attachment of a U-shaped clip to the portable unit of the smart entry system according to the first embodiment of the invention.
[Fig. 9] Fig. 9 is a flowchart showing how the smart entry system according to the first embodiment of the invention operates.
[Fig. 10] Fig. 10 is another flowchart showing how the smart entry system according to the first embodiment of the invention operates.
[Fig. 11] Figs. 11 (a) and 11 (b) show a portable unit of a smart entry system according to a second embodiment of the invention.
[Fig. 12] Fig. 12 is a flowchart showing how the smart entry system according to the second embodiment of the invention operates.
[Fig. 13] Fig. 13 is another flowchart showing how the smart entry system according to the first embodiment of the invention operates.
[Fig. 14] Figs. 14(a) and 14(b) show another example portable unit of the smart entry system according to the second embodiment of the invention.
[Fig. 15] Fig. 15 is a further flowchart showing how the smart entry system according to the first embodiment of the invention operates.
[Fig. 16] Fig. 16 shows a portable unit and a headset of a smart entry system according to a third embodiment of the invention.

### MODES FOR CARRYING OUT THE INVENTION

Materials employed in embodiments of the invention are just preferred examples and the usable materials are not limited to them. Modifications can be made as appropriate without departing from the scope of the concept of the invention. And each embodiment can be combined with another or other embodiments as long as no contradiction occurs.

### (Embodiment 1)

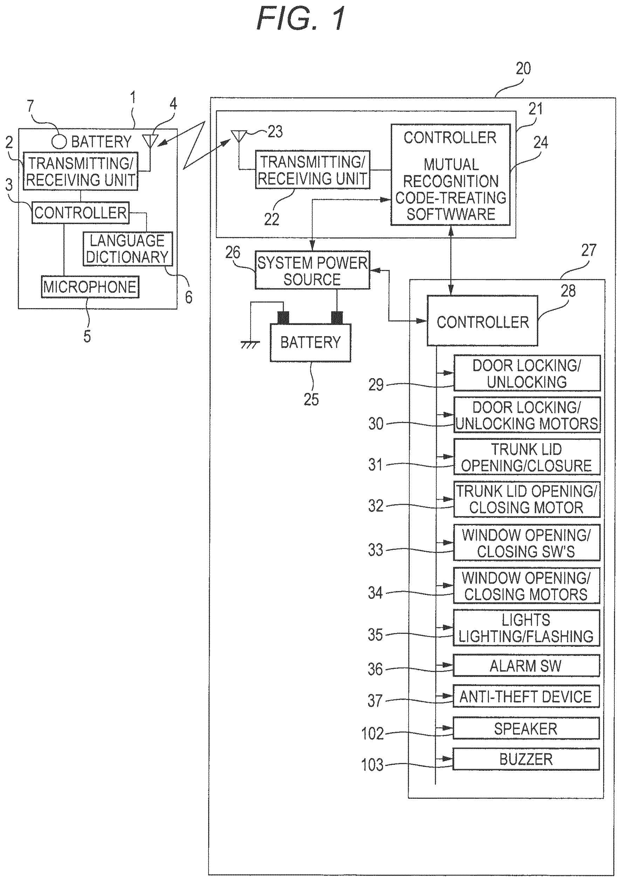

A first embodiment of the invention will be described below in detail with reference to Figs. 1-10.

Fig. 1 is a block diagram showing the configuration of a smart entry system according to the first embodiment of the invention. The smart entry system includes a portable unit 1 which a driver, for example, carries mainly outside the vehicle compartment, a vehicle-side smart entry device 21 provided in a vehicular apparatus 20 installed in a vehicle, a vehicle body control device 27 also provided in the vehicular apparatus 20, a battery 25, and a system power source 26.

The portable unit 1 includes a transmitting/receiving unit 2 and an antenna 4 for performing a wireless communication with the vehicle-side smart entry device 21, a controller 3, a microphone 5, a language dictionary 6, and a battery 7 as a power source of the portable unit 1. The language dictionary 6 is used for performing speech synthesis processing such as voice recognition on voice information that is picked up by the microphone 5,

The vehicle-side smart entry device 21 and the vehicle body control device 27 are supplied with power from the battery 25 via the system power source 26,

The vehicle-side smart entry device 21 includes a transmitting/receiving unit 22 and an antenna 23 for performing a wireless communication with the portable unit 1 and a controller 24 which incorporates mutual recognition code-treating software for performing authentication of a registered ID with the portable unit 1,

The vehicle body control device 27 includes a controller 28 for controlling (the operations of) door locking/unlocking 29, door opening/closing motors 30, trunk opening/closure (trunk lid opening/closure) 31 of a trunk lid which is a lock of a trunk, a trunk opening/closing motor 32, window opening/closing switches 33, a window opening/closing motors 34, lights lighting/flashing 35, an alarm SW (switch) 36, an anti-theft device 37, a speaker 102, and a buzzer 103.

The vehicle-side smart entry device 21 and the vehicle body control device 27 are electrically connected to each other and signals can be exchanged between them.

Each of the controllers 3, 24, and 28 is a microcomputer having, as main components, a CPU, a ROM, a RAM, a memory, an input/output unit (I/O), etc.

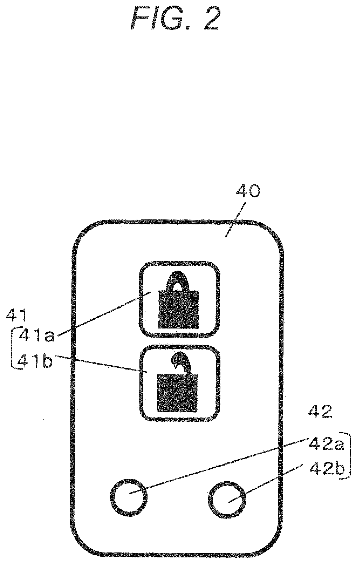

Fig. 2 is a front view showing an appearance of the portable unit of the smart entry system according to the first embodiment of the invention.

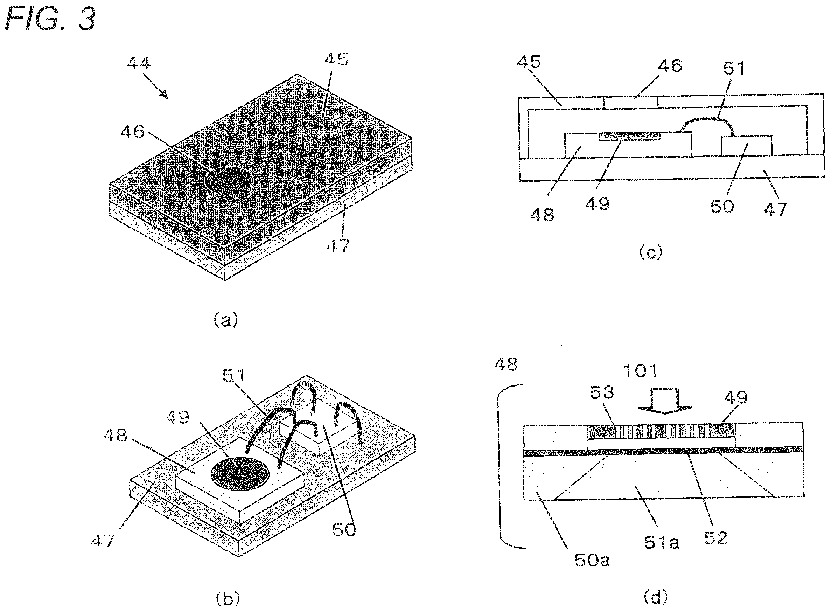

The portable unit 40 is provided with a switch group 41 consisting of a switch 41a for opening electromotive slide doors, for example, of the vehicle and a switch 41 b for closing the doors, and is formed with two holes (sound holes) 42a and 42b for picking up a voice of a talker such as the driver. Only one sound hole 42 may be provided. However, where speech synthesis processing for suppressing a wind noise originating from around the portable unit 40 is performed, it is desirable to provide two or more sound holes 42 because speech synthesis processing for noise suppression can be performed more effectively when plural sound conversion elements are used. Acoustic transducers as shown in Figs. 3(a)-3(d), such as electret capacitor microphones (ECMs), silicon microphones, or dynamic microphones, for converting the sound pressure of a voice or the like into an electric signal are disposed behind the sound holes 42 in the same number as the sound holes 42 in one-to-one correspondence.

Figs. 3(a)-3(d) are appearance diagrams showing an example acoustic transducer that is disposed inside the portable unit of the smart entry system according to the first embodiment of the invention. A silicon microphone is shown as an example acoustic transducer.

Fig. 3(a) is a schematic perspective view showing the configuration of the silicon microphone 44 which is an acoustic transducer. A metal cover 45 is placed on a circuit board 47 and is formed with a sound hole 46 through which a sound pressure is input.

Fig, 3(b) is a schematic perspective view, without the metal cover 45, showing the configuration of the silicon microphone 44. A MEMS chip 48 which is a sound conversion element and an IC chip 50 are mounted on the circuit board 47, and the MEMS chip 48 and the IC chip 50 are electrically connected to each other by bonding wires 51. The MEMS chip 48 is formed with a fixed film 49 having sound holes through which a sound pressure is input.

Fig. 3(c) is a sectional view showing the configuration of the silicon microphone 44. Fig. 3(c) shows that the sound hole 46 which is formed through the metal cover 45 and the fixed film 49 having the sound holes through which a sound pressure is input are disposed at such positions as to be opposed to each other.

Fig. 3(d) is a sectional view showing the configuration of the MEMS chip 48 which is a sound conversion element. The MEMS chip 48 is composed of a vibration film 52 which is formed on a silicon substrate 50a having a cavity 51a and the fixed film 49 having plural sound holes 53 through which a sound pressure is input. The vibration film 52 and the fixed film 49 have the function of a parallel-plate capacitor. As for the principle of operation of the MEMS chip 48 which functions as a sound conversion element, a phenomenon is utilized that the vibration film 52 is vibrated when a sound pressure (air vibration) 101 is transmitted to the vibration film 52 through the sound holes 53, whereby the gap (interval) between the vibration film 52 and the fixed film 49 is varied and hence the capacitance of the vibration film 52 and the fixed film 49 which constitute a parallel-plate capacitor is varied. The variation of the capacitance of the vibration film 52 and the fixed film 49 is electrically transmitted to the IC chip 50, which converts the capacitance variation into an electrical signal, amplifies the latter, and outputs a resulting electrical signal to the outside. The acoustic transducer (silicon microphone) 44 is formed in this manner.

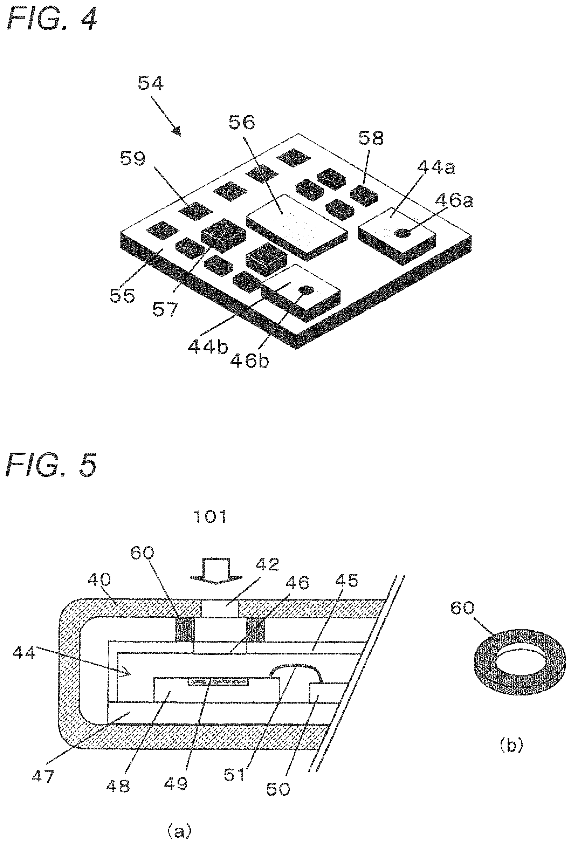

Fig. 4 is an appearance perspective view showing an example sound conversion module which is disposed inside the portable unit of the smart entry system according to the first embodiment of the invention. This example sound conversion module is provided with two silicon microphones (sound conversion elements).

In the sound conversion module 54 shown in Fig. 4, two acoustic transducers 44a and 44b are mounted on a circuit board 55. The acoustic transducers 44a and 44b are formed with respective sound holes 46a and 46b. An LSI 56 for performing speech synthesis processing, microcomputers 57 (two in the figure), and capacitors and resistors 58 are mounted on the circuit board 55 and perform electrical signal processing. Pads 59 for transmission of electrical signals to the outside are formed on the circuit board 55 and interconnections etc. (not shown) are connected to the pads 59, whereby power is input to the acoustic transducers 44 and electric signals as results of processing performed by the acoustic transducers 44 are transmitted outside. As for the functions of the sound conversion module 54, it performs signal processing such as noise suppression, speech synthesis, and voice recognition using the plural (in the embodiment, two) acoustic transducers (silicon microphones) 44. The sound conversion module 54 is disposed on the back wall of the portable unit 40 shown in Fig. 1 and the acoustic transducers 44 are disposed at such positions as to face the respective sound holes 42 of the portable unit 40.

Fig. 5(a) is a sectional view showing an example sound conversion module that is disposed inside the portable unit of the smart entry system according to the first embodiment of the invention, and Fig. 5(b) is an appearance perspective view of a sound shield member.

Fig. 5(a) is a sectional view of the portable unit 40, and shows that the fixed film 49 formed in one MEMS chip 48 and having the sound holes through which a sound pressure is input is disposed so as to face the sound hole 42 formed in the portable unit 40 and the sound hole 46 formed through the metal cover 45. A speaking sound pressure 101 of the driver, for example, is input from above the sound hole 42. The sound shield member 60 is sandwiched between the portable unit 40 and the metal cover 45 and prevents the sound pressure 101 from diffusing inside the portable unit 40. If diffusing inside the portable unit 40 which has a large volume, the sound pressure 101 causes a resonance phenomenon inside the portable unit 40 to deteriorate the acoustic characteristics of the acoustic transducers (silicon microphones) 44. The sound shield member 60 is disposed so as to prevent deterioration of the acoustic characteristics of the acoustic transducers 44. Fig. 5(b) is an appearance perspective view of the sound shield member 60. The sound shield member 60 employed in the embodiment assumes a hollow, doughnut shape, and its external shape need not always be circular and may be rectangular, octagonal, or of a like shape.

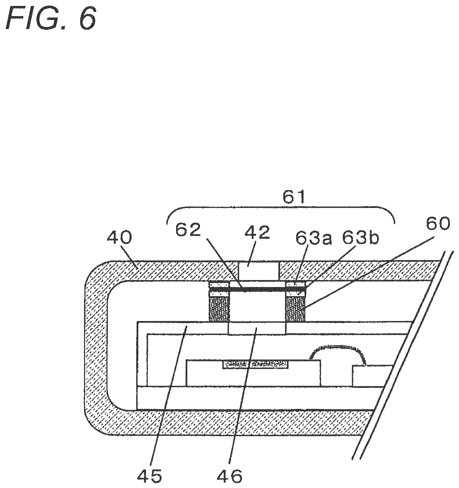

Fig. 6 is a sectional view showing another example sound conversion module that is disposed inside the portable unit of the smart entry system according to the first embodiment of the invention. This sound conversion module is different from the one shown in Fig. 5(a) in that a water-proof structural body 61 is interposed between the portable unit 40 and each sound shield member 60. The water-proof structural body 61 is composed of a water-proof film 62 and adhesive members 63 (denoted by symbols 63a and 63b in the figure). It is preferable that the material of the water-proof film 62 be stretched PTFE, which has a porous PTFE structure that is formed with a large number of minute holes called pores. Porous PTFE formed with a large number of minute holes has features that it transmits a sound pressure while prevents permeation of water even if water droplets such as raindrops attach to it. Therefore, when a user of the portable unit 40 such as the driver uses the portable unit 40 outdoors on a rainy day, no failure occurs in the sound conversion elements even if the portable unit 40 gets wet. The convenience of the user can thus be increased.

Although in Fig. 6 the water-proof structural body 61 is interposed between the portable unit 40 and each sound shield member 60, the sound shield member 60 may be disposed on the top side or the back side of the sound hole 46 formed through the metal cover 45 of each acoustic transducer 44 or on the top side of each sound hole 42 of the portable unit 40.

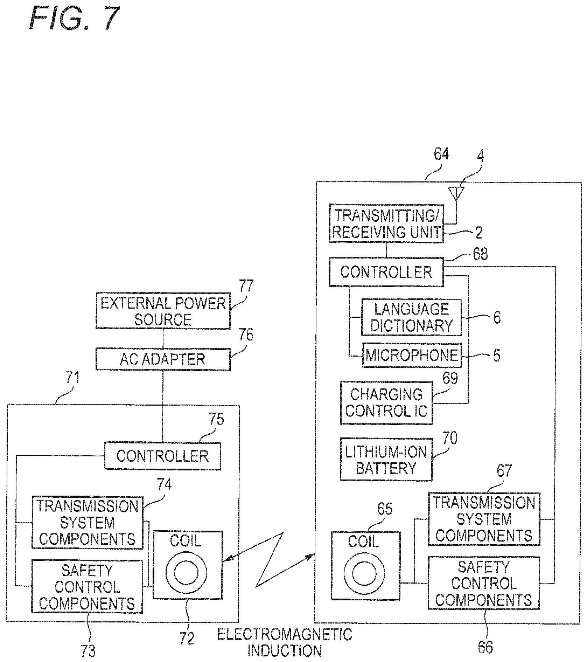

Fig. 7 is a schematic block diagram showing a mechanism for charging the portable unit of the smart entry system according to the first embodiment of the invention by non-contact charging. Fig. 7 shows a configuration capable of charging the portable unit 64 when it is placed on or brought close to a charging stage 71. Charging is done by causing electromagnetic induction between the portable unit 64 and the charging stage 71. More specifically, when a current is caused to flow through one of two adjoining coils, a resulting magnetic flux causes generation of an electromotive force in the other coil, whereby charging is done.

The portable unit 64 shown in Fig. 7 includes, in addition to the transmitting/receiving unit 2 etc. shown in Fig. 1, a coil 65 for electromagnetic induction, safety control components 66, transmission system components 67, a controller 68, a charging control IC 69, and a lithium-ion battery 70. Power that is generated in the coil 65 by an electromotive force via a magnetic flux generated by causing a current to flow through the charging stage 71 is transmitted to the controller 68 via the safety control components 66 and the transmission system components 67. Thus, the controller 68 can store power in the lithium-ion battery 70 via the charging control IC 69. The charging stage 71 includes a coil 72 for causing electromagnetic induction, safety control components 73, transmission system components 74, and a controller 75. Power is supplied from an external power source 77 to the controller 75 via an AC adapter 76. When a current is caused to flow through the coil 72 under the control of the controller 75, the portable unit 64 is charged in the above-described manner.

The portable unit shown in Fig. 1 which uses a general-purpose battery may suffer a system shutdown due to sudden running-out of the battery of the portable unit. A sudden system shutdown can be prevented by giving the portable unit 64 a charging function in the manner shown in Fig. 7 because a user can charge it any time when necessary. If the portable unit 64 has a function of displaying information to the effect that charging is necessary (e.g., lighting of a red LED), a user can recognize that charging of the portable unit 64 is necessary, whereby charging of the portable unit 64 is urged. The addition of this function increases the convenience of a user.

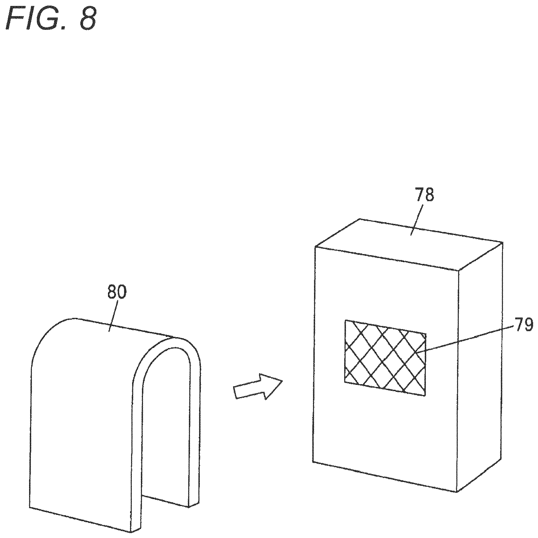

Fig. 8 shows an example manner of attachment of a U-shaped clip to the portable unit of the smart entry system according to the first embodiment of the invention.

Fig. 8 shows the portable unit 78 of the smart entry system, a magnet 79 which is buried in the rear wall of the portable unit 78, and the U-shaped clip 80 made of a metal. In the example of Fig. 8, the U-shaped clip 80 is separable from the portable unit 78 and is attached to and detached from the portable unit 78 when necessary because of the presence of the magnet 79. That is, the U-shaped clip 80 is detachable. A portable unit with a detachable U-shaped clip is more convenient than a portable unit that is integrated with a U-shaped clip, for the following reason. If a portable unit that is integrated with a U-shaped clip is put in a trouser pocket or a bag, the pocket or the bag is rendered bulky. In contrast, a portable unit with a detachable U-shaped clip does not render a pocket or bag bulky while it is being carried. The convenience of the user is thus increased.

Although the example of Fig. 8 uses the U-shaped clip, the invention is not limited to the case of using a U-shaped clip and a tie-pin-shaped clip (not shown) may be used instead.

A portable unit that is equipped with a tie-pin-shaped clip, instead of a U-shaped clip, increases the convenience of use of the smart entry system very much because it can be attached to a chest pocket. There are plural reasons for this. For example, since the portable unit is set close to the mouth of a user, a voice spoken by the user can be picked up better, whereby the recognition rate of voice recognition by the portable unit is increased. Furthermore, when a portable unit is put in a pocket or a bag, the portable unit rubs against, for example, a cloth portion of the pocket or the bag to produce a rustle sound or a friction sound, which becomes a noise at the time of sound picking-up by a microphone and lowers the recognition rate of voice recognition by the portable unit. This phenomenon can be avoided.

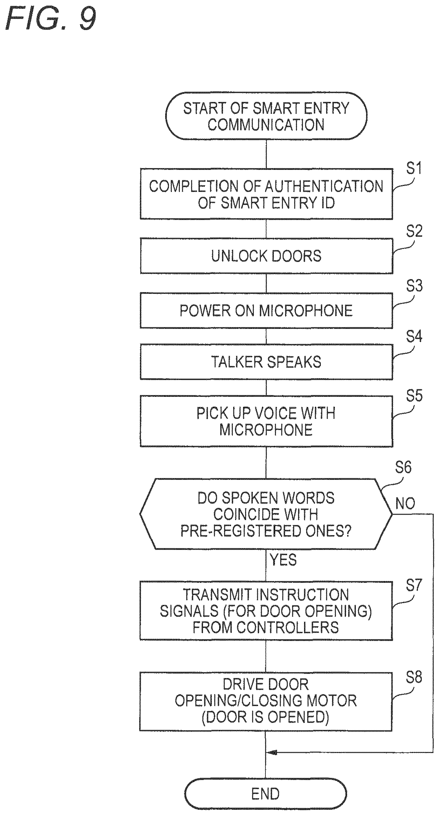

Fig. 9 is a flowchart showing how the smart entry system according to the first embodiment of the invention operates. More specifically, Fig. 9 is a flowchart of an operation of opening an electromotive slide door of the vehicle in a handsfree manner.

First, when the driver, for example, who has put the portable unit of the smart entry system in, for example, a chest pocket approaches the vehicle, a communication between the portable unit and the vehicle-side smart entry device is started. When ID authentication in the smart entry system has finished at step S1, at step S2 the door locking of the electromotive slide doors is canceled and the doors are unlocked. At step S3, the microphone (the sound conversion module 54 shown in Fig. 4) that is provided in the portable unit is powered on. At step S4, when the driver, for example, speaks predetermined words, say, "Open the rear-right door!", near the microphone, at step S5 the microphone picks up the voice spoken. The predetermined words function as a voice key. At step S6, it is verified whether or not the spoken words coincide with pre-registered ones. If the spoken words coincide with pre-registered ones, the process moves to step S7. At step S7, the controllers 24 and 28 in the vehicular apparatus 20 shown in Fig. 1 transmit opening signals for the electromotive slide door concerned. At step S8, the door opening/closing motor 30 is driven and the electromotive slide door is opened. On the other hand, if the spoken words do not coincide with any pre-registered ones, the process is finished immediately.

If an electromotive slide door of a vehicle can be opened and closed in a handsfree manner, it becomes possible for a mother holding her baby to open a door without the need for touching the vehicle or for the driver, for example, raising an umbrella to open a door without the need for touching the vehicle. Thus, the convenience of use of the automobile is increased very much.

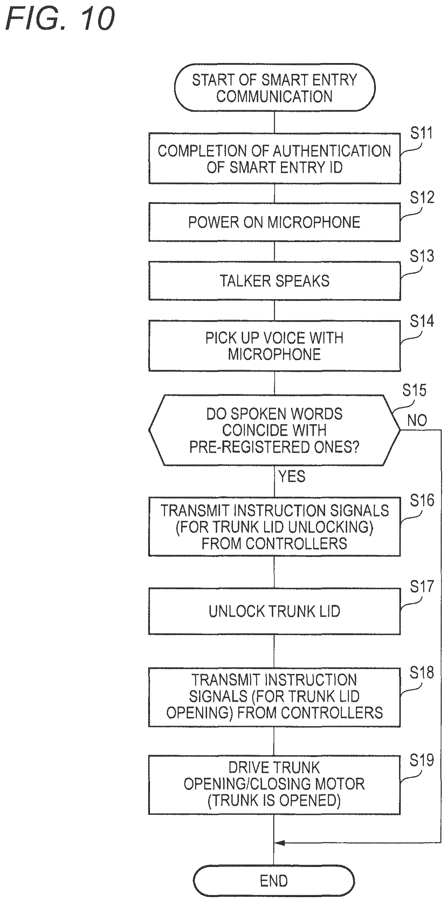

Fig. 10 is another flowchart showing how the smart entry system according to the invention operates. More specifically, Fig. 10 is a flowchart of an operation of opening the trunk of the vehicle in a handsfree manner, which is different from the flowchart of Fig. 9.

Vehicle trunks are not opened even if a smart entry device comes close to them. This is because it is not necessary to open the vehicle truck every time a smart entry device comes close to it. The driver, for example, can open the trunk by pushing a trunk opening/closing button that is disposed outside the trunk even if he or she has a smart entry device. However, to open the trunk when the driver, for example, is holding a big load such as a golf bag with both hands and hence neither of his or her bands is usable, he or she needs to take the trouble to let the load off, which is inconvenient.

In the flowchart of Fig. 10, first, when the driver, for example, who has put the portable unit of the smart entry system in, for example, a chest pocket approaches the vehicle, a communication between the portable unit and the vehicle-side smart entry device is started. When ID authentication in the smart entry system has finished at step S11, at step S12 the microphone (the sound conversion module 54 shown in Fig. 4) that is provided in the portable unit is powered on. At step S13, when the driver, for example, speaks predetermined words, say, "Open the trunk!", near the microphone, at step S14 the microphone picks up the voice spoken. The predetermined words function as a voice key. At step S15, it is verified whether or not the spoken words coincide with pre-registered ones. If the spoken words coincide with pre-registered ones, the process moves to step S16. At step S16, the controllers 24 and 28 in the vehicular apparatus 20 shown in Fig. 1 transmit unlocking signals for the trunk lid. At step S17, the trunk lid is unlocked. At step S18, the controllers 24 and 28 in the vehicular apparatus 20 shown in Fig. 1 commands driving of the trunk opening/closing motor 32. At step S19, t trunk opening/closing motor 32 is driven and the trunk lid is opened. On the other hand, if the spoken words do not coincide with any pre-registered ones, the process is finished immediately.

If the trunk of a vehicle can be opened and closed in a handsfree manner, it becomes possible for the driver, for example, who is holding a big load to open the trunk without the need for touching the vehicle. Thus, the convenience of use of the automobile is increased very much. Furthermore, if a backdoor can be opened and closed in a handsfree manner, in allowing a wheelchair user who is disabled in the legs to get on an automobile from its back side (trunk side) in a state that he or she is sitting in the wheelchair, it becomes possible to reduce the burden of a wheelchair helper. Thus, the convenience of use of the automobile is increased very much.

In the flowcharts of Figs. 9 and 10 which show how the smart entry system operates, predetermined words function as a voice key but no discrimination is made between a particular talker and an indefinite talker for the voice key. Applying a voice recognition technique in which reaction is made only to a particular talker to the voice key used in the above flowcharts is helpful to, for example, automobile theft prevention because a handsfree personal authentication function is thereby added.

### (Embodiment 2)

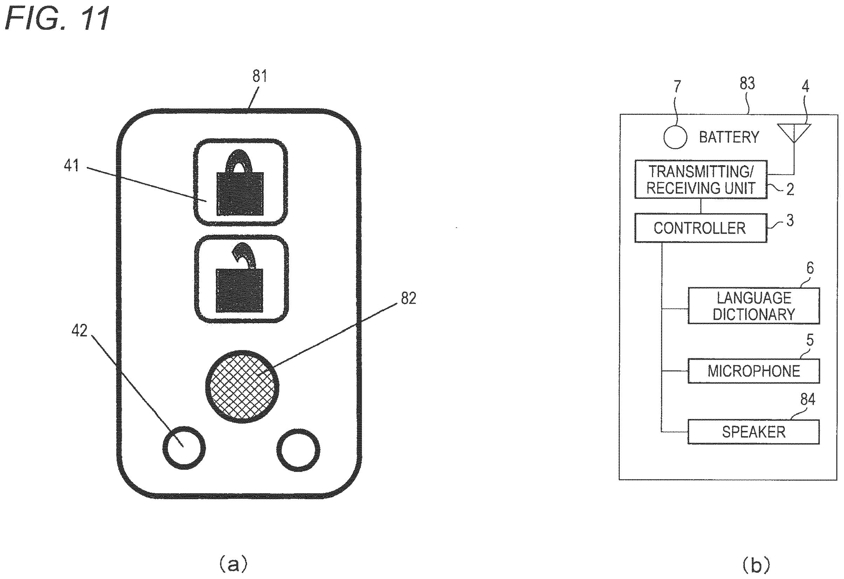

A second embodiment of the invention will be described below in detail with reference to Figs. 11(a)-15.

Figs. 11 (a) and 11(b) show a portable unit of a smart entry system according to the second embodiment of the invention. The portable unit employed in this embodiment is different from the portable unit according to the first embodiment shown in Figs. 1 and 2 in that the former is provided with a speaker.

In this embodiment, the portable unit shown in Fig. 11(a) includes a speaker 82. Fig. 11(b) also shows that the portable unit 83 includes the speaker 84. Equipping the portable unit with the speaker increases the convenience of the smart entry system further.

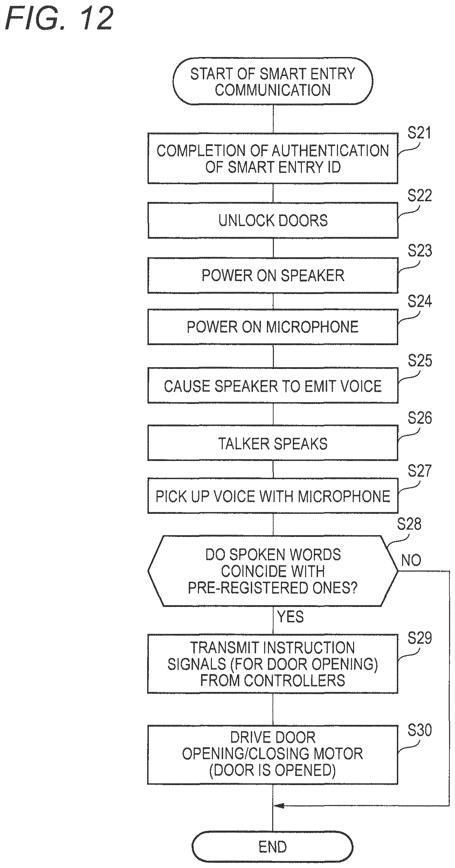

Fig. 12 is a flowchart showing how the smart entry system according to the second embodiment of the invention operates. This is a flowchart of an operation of opening an electromotive slide door of the vehicle in a handsfree manner. This flowchart is different from the flowchart shown in Fig. 9 in that a step of powering on a speaker (step S23 in Fig. 12) and a step of causing the speaker to emit a sound (step S25 in Fig. 12) are added.

In the flowchart shown in Fig. 12, first, when the driver, for example, who has put the portable unit of the smart entry system in, for example, a chest pocket approaches the vehicle, a communication between the portable unit and the vehicle-side smart entry device is started. When ID authentication in the smart entry system has finished at step S21, at step S22 the door locking of the electromotive slide doors is canceled and the doors are unlocked. The speaker is powered on at step S23 and the microphone (the sound conversion module 54 shown in Fig. 4) which is disposed in the portable unit is powered on at step S24. At step S25, predetermined words, say, "Welcome back!", are emitted from the speaker 82 (84) which is provided in the portable unit. This serves to urge a talker such as the driver to speak. At step S26, when the driver, for example, speaks predetermined words, say, "Open the rear-right door!", near the microphone, at step S27 the microphone picks up the voice spoken. The predetermined words function as a voice key. At step S28, it is verified whether or not the spoken words coincide with pre-registered ones. If the spoken words coincide with pre-registered ones, the process moves to step S29. At step S29, the controllers 24 and 28 in the vehicular apparatus 20 shown in Fig. 1 transmit opening signals for the electromotive slide door concerned. At step S30, the door opening/closing motor 30 is driven and the electromotive slide door is opened. On the other hand, if the spoken words do not coincide with any pre-registered ones, the process is finished immediately.

In the embodiment, since the function of urging a talker such as the driver to speak is provided, a dialogue-type scheme is established which allows the talker to speak with good timing. Thus, the convenience of the talker is increased.

Although in the embodiment a talker is urged to speak by the speaker which is provided in the portable unit, the same advantage is obtained by urging a talker to speak even by the speaker (the speaker 102 shown in Fig. 1) provided on the vehicular apparatus side. It is also possible to urge a talker to speak by emitting a buzzer sound using the buzzer (the buzzer 103 shown in Fig. 1) provided on the vehicular apparatus side or to urge a talker to speak by lighting or flashing lights provided on the vehicular apparatus side (lights lighting/flashing 35 shown in Fig. 1).

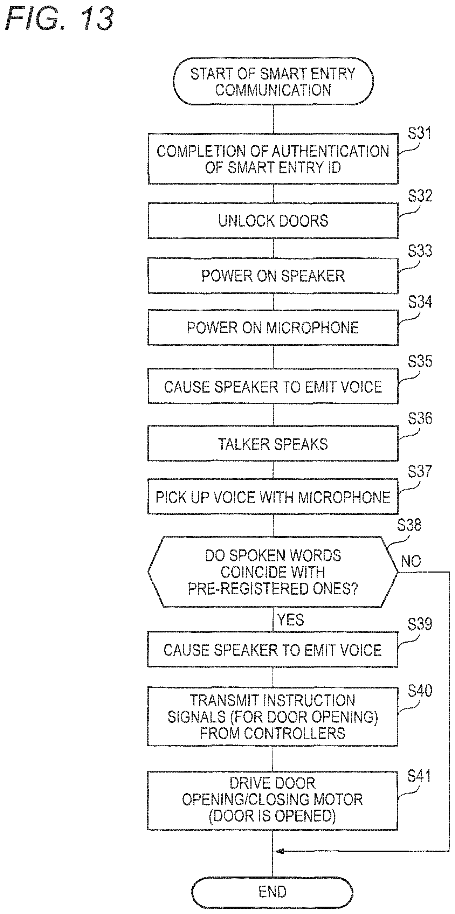

Fig. 13 is another flowchart showing how the smart entry system according to the second embodiment of the invention operates. This is a flowchart of an operation of opening an electromotive slide door of the vehicle in a handsfree manner. This flowchart is different from the flowchart shown in Fig. 12 in that another step of causing the speaker to emit a voice (step S39 in Fig. 13) is added.

Steps S31-S38 of the flowchart shown in Fig. 13 are the same as steps S21-S28 of the flowchart shown in Fig. 12. Step S39 shown in Fig. 13 is a step at which when spoken words coincide with pre-registered words (S38: yes), the talker is notified of the fact that the voice key of the talker has been acknowledged using the microphone that is provided in the portable unit by causing the speaker to emit a word "Acknowledged!" The talker is relieved to hear the response "Acknowledged!" Thus, a further advanced form of dialogue-type conversation is realized. The subsequent steps S40 and 41 are the same as steps S29 and S30. The same advantage as obtained by causing the speaker to emit the word "Acknowledged!" can be obtained by blowing a buzzer sound or lighting or flashing lights.

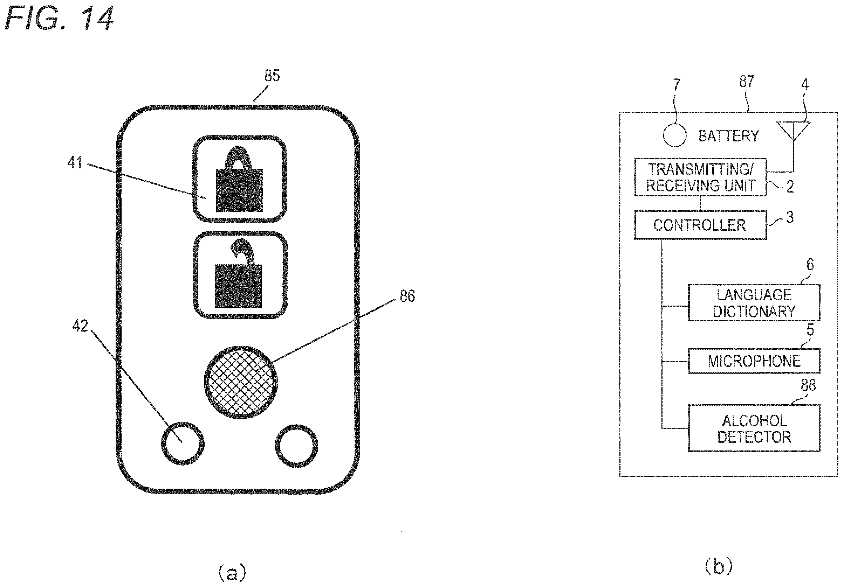

Figs. 14(a) and 14(b) shows another example portable unit of the smart entry system according to the second embodiment of the invention. The portable unit employed in this embodiment is different from the portable unit according to the first embodiment shown in Figs. 1 and 2 in that the former is provided with an alcohol detector.

In this embodiment, the portable unit 85 shown in Fig. 14(a) includes an alcohol detector 86. Fig. 14(b) also shows that the portable unit 87 includes the alcohol detector 88. Equipping the portable unit with the alcohol detector increases the convenience of the smart entry system further. More specifically, this makes it possible to suppress drunken driving.

Example types of the alcohol detector include a semiconductor type, a fuel cell type, a non-dispersive infrared (NDIR) type, and a chemical reaction type. However, in the invention, there are no limitations on the alcohol detector type; for example, a type that is advantageous in terms of miniaturization may be selected and used.

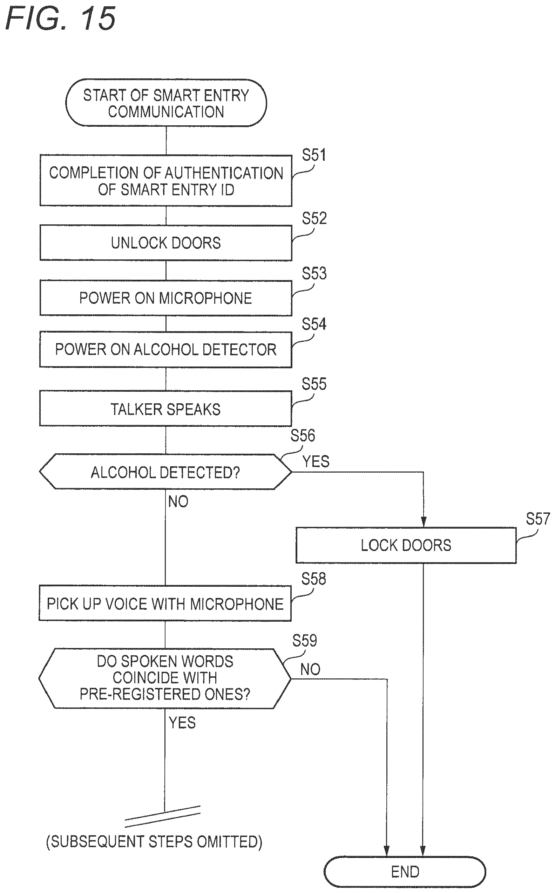

Fig. 15 is a further flowchart showing how the smart entry system according to the second embodiment of the invention operates. This is a flowchart of an operation of stopping the smart entry system when alcohol is detected from a talker in opening an electromotive slide door of the vehicle in a handsfree manner.

In the flowchart shown in Fig. 15, first, when the driver, for example, who has put the portable unit of the smart entry system in, for example, a chest pocket approaches the vehicle, a communication between the portable unit and the vehicle-side smart entry device is started. When ID authentication in the smart entry system has finished at step S51, at step S52 the door locking of the electromotive slide doors is canceled and the doors are unlocked. The microphone is powered on at step S53 and the alcohol detector is powered on at step S54. At step S55, when the driver, for example, speaks predetermined words, say, "Open the rear-right door!", near the microphone, at step S56 it is judged whether or not expiratory air contains alcohol using the alcohol detector. If alcohol is detected, the process moves to step S57, where the once unlocked doors are locked. Then the process is finished. On the other hand, if no alcohol is detected, the process moves to step S58, where the microphone picks up the voice spoken. At step S59, it is verified whether or not the spoken words coincide with pre-registered ones. If the spoken words coincide with pre-registered ones, the process moves to the next step (a description of the subsequent steps will be omitted). On the other hand, if the spoken words do not coincide with any pre-registered ones, the process is finished immediately.

Although not shown in Fig. 15, a function of notifying the driver, for example, that expiratory air contains alcohol by disposing a speaker in the portable unit or using the speaker provided in the vehicle may be provided.

According to the embodiment, if alcohol is detected in expiratory air of, for example, the driver, he or she is not allowed to enter the inside of the automobile and hence cannot drive it. Thus, drunken driving can be suppressed.

### (Embodiment 3)

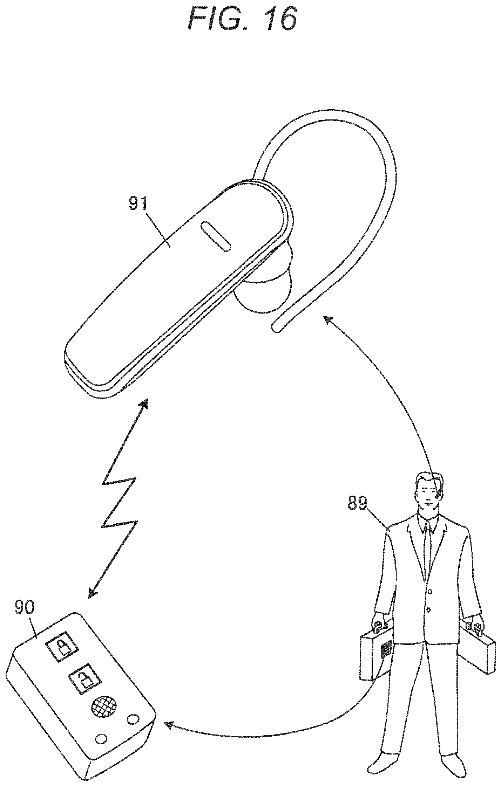

A third embodiment of the invention will be described below in detail with reference to Fig. 16. Fig. 16 shows a portable unit and a headset of a smart entry system according to the third embodiment of the invention.

A person 89 (e.g., driver) who is carrying loads and neither of whose hands is usable is shown in Fig. 16. The person 89 has put a portable unit 90 in one load (e.g., bag) and wears a headset 91 around his ear. Whereas the portable unit 90 has the same functions as the portable unit employed in the first or second embodiment, the former is different from the latter in being capable of performing a wireless communication with the headset 91 according to Bluetooth (registered trademark), for example. The headset 91 is equipped with, in addition to a wireless communication device for communication with the portable unit 90, a microphone (sound conversion module), an earphone (speaker), and an alcohol detector. The earphone (speaker) and the alcohol detector may be omitted.

In the configuration shown in Fig. 16, since the portable unit 90 is put in the load (e.g., bag), a noise such as a friction sound is produced by friction between the portable unit 90 and cloth located inside the load. Therefore, even if a person such as a driver spoke to generate a voice key, it would be difficult to recognize the voice key that is buried in the noise using the microphone (sound conversion module) of the portable unit 90.

In contrast, where a voice key is acquired using the headset 91 shown in Fig. 16 and that information is transmitted by radio, a satisfactory handsfree smart entry system can be provided that is free of any restrictions relating to an installation environment of the portable unit 90.

Where a sound shield member is disposed in front of each sound conversion element that is provided in the headset, as described in the first embodiment, entrance of a disturbance sound pressure can be prevented by the sound shield member. Thus, it becomes possible to transmit a necessary sound pressure of a voice or the like to the sound conversion element efficiently.

Where a water-proof film or a planar fabric having a water-repelling function is disposed in front of each sound hole formed in the headset, entrance of water droplets etc. can be prevented and hence the headset can be prevented from failing due to entrance of water.

It goes without saying that also in this embodiment, as in the first embodiment, it is possible to make effective a predetermined, particular spoken voice as a voice key only when it is spoken by a particular talker, that is, handsfree personal authentication is realized.

Although the invention has been described in detail by referring to the particular embodiments, it is apparent to those skilled in the art that various changes and modifications are possible without departing from the spirit and scope of the invention.

The present application is based on Japanese Patent Application No. 2013-233185 filed on November 11, 2013.

### Industrial Applicability

The invention is useful when applied to smart entry systems that require picking-up of a voice of a person who is speaking outside a vehicle.

### Description of Symbols

1: Portable unit
3: Controller
5: Microphone
20: Vehicular apparatus
40: Portable unit
42: Sound hole
44: Acoustic transducer (silicon microphone)
54: Sound conversion module
60: Sound shield member
61: Water-proof structural body
80: U-shaped clip
82, 84: Speaker
86, 88: Alcohol detector
91: Headset

## Claims

1. A smart entry system comprising:
a portable unit (90) configured to transmit and receive a signal; and
a vehicular apparatus (20) installed in a vehicle;
**characterized by**
a headset (91) configured to perform a wireless communication with the portable unit (90), and to perform at least part of locking controls and unlocking controls on the vehicle by wirelessly communicating authentication signals between the portable unit (90) and the vehicular apparatus (20) bidirectionally,
wherein the headset (91) comprises:
at least one hole through which a sound pressure is input;
a sound conversion element which is disposed behind the hole and converts the sound pressure into an electrical signal; and
a voice recognition device configured to process the electrical signal obtained by the conversion using a predetermined particular spoken voice as a voice key;
wherein the headset (91) transmits an input signal of the voice key to the portable unit (90) by a wireless communication; and
wherein the portable unit (90) instructs the vehicular apparatus (20) on at least part of operations of the vehicle by radio.

2. The smart entry system according to claim 1, wherein the instruction on the at least part of the operations of the vehicle is an opening/closure instruction for a door of the vehicle.

3. The smart entry system according to claim 1, wherein the instruction on the at least part of the operations of the vehicle is an opening/closure instruction for a trunk of the vehicle.

4. The smart entry system according to any one of claims 1 to 3, wherein a speaker is provided in the headset (91); and
wherein the smart entry system comprises a function of urging a talker (89) to speak by the speaker.

5. The smart entry system according to any one of claims 1 to 4, wherein a sound shield member is interposed between the hole of the headset (91) and the sound conversion element.

6. The smart entry system according to any one of claims 1 to 5, wherein a water-proof film or a planar fabric having a water-repelling function is disposed in front of the hole of the headset (91).

7. The smart entry system according to any one of claims 1 to 6, further comprising:
an alcohol detector provided in the headset (91),
wherein the smart entry system comprises a function of disregarding a spoken voice of any voice key if the alcohol detector detects alcohol.

8. The smart entry system according to any one of claims 1 to 7, wherein the predetermined particular spoken voice which is used as the voice key is made effective only when spoken by a particular talker (89).

## Patentansprüche

1. Smart-Entry-System, das umfasst:
eine tragbare Einheit (90), die zum Senden und Empfangen eines Signals eingerichtet ist; sowie
eine Fahrzeug-Vorrichtung (20), die in einem Fahrzeug installiert ist;
**gekennzeichnet durch**
ein Headset (91), das zum Durchführen einer Drahtlos-Kommunikation mit der tragbaren Einheit (90) sowie zum Durchführen wenigstens eines Teils von Verriegelungs-Bedienvorgängen und Entriegelungs-Bedienvorgängen an dem Fahrzeug mittels bidirektionaler drahtloser Übertragung von Authentifizierungs-Signalen zwischen der tragbaren Einheit (90) und der Fahrzeug-Vorrichtung (20) eingerichtet ist,
wobei das Headset (91) umfasst:
wenigstens ein Durchgangsloch, über das ein Schalldruck eingeleitet wird;
ein Schallumwandlungs-Element, das hinter dem Loch angeordnet ist und den Schalldruck in ein elektrisches Signal umwandelt; und
eine Spracherkennungs-Einrichtung, die so eingerichtet ist, dass sie das mittels der Umwandlung gewonnene elektrische Signal unter Verwendung einer vorgegebenen bestimmten Sprechstimme als einem Sprach-Schlüssel (voice key) verarbeitet;
wobei das Headset (91) ein Eingangssignal des Sprach-Schlüssels mittels einer Drahtlos-Kommunikation zu der tragbaren Einheit (3) sendet; und
die tragbare Einheit (90) die Fahrzeug-Vorrichtung (20) bezüglich wenigstens eines Teils von Funktionen des Fahrzeugs über Funk anweist.

2. Smart-Entry-System nach Anspruch 1, wobei die Anweisung bezüglich wenigstens eines Teils der Funktionen des Fahrzeugs eine Anweisung zum Öffnen/Schließen einer Tür des Fahrzeugs ist.

3. Smart-Entry-System nach Anspruch 1, wobei die Anweisung bezüglich wenigstens eines Teils der Funktionen des Fahrzeugs eine Anweisung zum Öffnen/Schließen eines Kofferraums des Fahrzeugs ist.

4. Smart-Entry-System nach einem der Ansprüche 1 bis 3, wobei in dem Headset (91) ein Lautsprecher vorhanden ist; und
das Smart-Entry-System eine Funktion umfasst, durch die ein Sprecher (89) über den Lautsprecher aufgefordert wird zu sprechen.

5. Smart-Entry-System nach einem der Ansprüche 1 bis 4, wobei ein Schall-Abschirmungselement zwischen dem Loch des Headsets (91) und dem Schallumwandlungs-Element angeordnet ist.

6. Smart-Entry-System nach einem der Ansprüche 1 bis 5, wobei eine wasserdichte Folie oder ein planes Gewebe, das eine Wasserabweisungs-Funktion aufweist, vor dem Loch des Headsets (91) angeordnet ist.

7. Smart-Entry-System nach einem der Ansprüche 1 bis 6, das des Weiteren umfasst:
einen Alkohol-Detektor, der sich in dem Headset (91) befindet,
wobei das Smart-Entry-System eine Funktion umfasst, durch die eine Sprechstimme eines Sprach-Schlüssels ignoriert wird, wenn der Alkohol-Detektor Alkohol erfasst.

8. Smart-Entry-System nach einem der Ansprüche 1 bis 7, wobei die vorgegebene bestimmte Sprechstimme, die als der Sprach-Schlüssel verwendet wird, nur wirksam wird, wenn sie von einem bestimmten Sprecher (89) gesprochen wird.

## Revendications

1. Système d'entrée intelligent comprenant :
une unité portable (90) configurée pour transmettre et recevoir un signal, et
un appareil de véhicule (20) installé dans un véhicule,
**caractérisé par**
un casque d'écoute (91) configuré pour effectuer une communication sans fil avec l'unité portable (90) et pour effectuer au moins une partie de commandes de verrouillage et de déverrouillage sur le véhicule en transmettant sans fil dans les deux sens des signaux d'authentification entre l'unité portable (90) et l'appareil de véhicule (20),
dans lequel le casque d'écoute (91) comprend :
au moins un trou au travers duquel une pression sonore est appliquée en entrée,
un élément de conversion de son qui est disposé derrière le trou et convertit la pression sonore en un signal électrique, et
un dispositif de reconnaissance de la voix configuré pour traiter le signal électrique obtenu par la conversion en utilisant une voix parlée particulière prédéterminée en tant que voix clé,
dans lequel le casque d'écoute (91) transmet un signal d'entrée de la voix clé à l'unité portable (90) grâce à une communication sans fil, et
dans lequel l'unité portable (90) ordonne à l'appareil de véhicule (20) au moins une partie des opérations du véhicule par radio.

2. Système d'entrée intelligent selon la revendication 1, dans lequel l'instruction sur la ou les parties des opérations du véhicule est une instruction d'ouverture / fermeture pour une portière du véhicule.

3. Système d'entrée intelligent selon la revendication 1, dans lequel l'instruction sur la ou les parties des opérations du véhicule est une instruction d'ouverture / fermeture pour un coffre du véhicule.

4. Système d'entrée intelligent selon l'une quelconque des revendications 1 à 3, dans lequel un haut-parleur est utilisé dans le casque d'écoute (91), et
le système d'entrée intelligent comprend une fonction de sollicitation d'un locuteur (89) pour parler au travers du haut-parleur.

5. Système d'entrée intelligent selon l'une quelconque des revendications 1 à 4, dans lequel un élément de blindage sonore est intercalé entre le trou du casque d'écoute (91) et l'élément de conversion de son.

6. Système d'entrée intelligent selon l'une quelconque des revendications 1 à 5, dans lequel un film étanche à l'eau ou un tissu plan comportant une fonction hydrofuge est placé en face du trou du casque d'écoute (91).

7. Système d'entrée intelligent selon l'une quelconque des revendications 1 à 6, comprenant en outre :
un détecteur d'alcool prévu dans le casque d'écoute (91),
le système d'entrée intelligent comprenant une fonction de rejet d'une voix parlée appartenant à n'importe quelle voix clé si le détecteur d'alcool détecte de l'alcool.

8. Système d'entrée intelligent selon l'une quelconque des revendications 1 à 7, dans lequel la voix parlée particulière prédéterminée qui est utilisée en tant que voix clé est rendue effective uniquement lorsqu'elle est parlée par un locuteur particulier (89).
